# EUROPEAN PATENT SPECIFICATION

(11) **EP 2 263 626 B1**
(45) Date of publication and mention of the grant of the patent: **07.05.2014**
(21) Application number: 09727574.7
(22) Date of filing: 31.03.2009
(51) Int. Cl.: A61F 13/15, A61F 13/49, A61F 13/56

(54) **MANUFACTURING METHOD FOR ABSORBENT PRODUCTS AND MANUFACTURING DEVICE FOR ABSORBENT PRODUCTS**
VERFAHREN UND VORRICHTUNG ZUR HERSTELLUNG SAUGFÄHIGER PRODUKTE
PROCÉDÉ DE FABRICATION DE PRODUITS ABSORBANTS ET DISPOSITIF DE FABRICATION DE PRODUITS ABSORBANTS

(30) Priority: 31.03.2008 JP 2008094112; 25.03.2009 JP 2009074770
(43) Date of publication of application: 22.12.2010
(73) Proprietor: Unicharm Corporation, Ehime 799-0111 (JP)
(72) Inventor: SAKAGUCHI, Satoru, Kanonji-shi Kagawa 769-1602 (JP); KAMEDA, Noritomo, Kanonji-shi Kagawa 769-1602 (JP); OKU, Tomomi, Kanonji-shi Kagawa 769-1602 (JP)
(74) Representative: Eke, Philippa Dianne
(86) International application number: PCT/JP2009/056621
(87) International publication number: WO 2009/123177

(56) References cited:
- WO-A1-2007/004640
- JP-A- 58 087 303
- JP-T- 2000 510 349
- JP-T- 2005 503 892
- JP-U- 59 165 404
- US-A1- 2004 188 004

## Description

### TECHNICAL FIELD

The present invention relates to a manufacturing method and a manufacturing apparatus for absorbent articles, each of which includes: a liquid permeable top sheet, a liquid impermeable back sheet and an absorber body that is interposed between the top sheet and the back sheet.

### BACKGROUND ART

In general, in order to be easily putted on the wearer, an open-type diaper needs to be provided with side flaps (projected parts) projected outwardly from an absorbent body.

Heretofore, for providing such side flaps, a following method has been known; that is, forming side panel members projecting outwardly from the absorbent body, and thereafter trimming the side panel members so that remaining parts thereof can serve as side flaps. However, this method has a problem in which many materials go wasted because of the trimming.

As a method for solving this problem, a manufacturing method for a diaper disclosed in Patent Literature 1 is known. The manufacturing method for this diaper includes the following steps:
(1) forming a diaper main body having a belt shape;
(2) forming a sheet having a belt shape into side flap sheets each having an approximately trapezoidal shape;
(3) bonding the side flap sheets to both side edges of the diaper main body having the belt shape; and
(4) cutting the diaper main body having the belt shape and the side flap sheets to predetermined dimensions.
Patent Literature 1: Japanese Patent Application Publication No. 04-261655

However, in the above manufacturing method for the open-type diapers, the side flap sheets need to be accurately positioned and bonded to the predetermined position of both side edges of each of the diaper main bodies each having the belt shape. Accordingly, this method has a problem of requiring much control and thus requires a sophisticated manufacturing facility.

In addition, in the above manufacturing method for open-type diapers, the side flap sheets placed on each diapers are different between the right side and the left side of each of the diapers. Accordingly, there has been a problem that bonding the side flap sheets is complicating when the position of the side flap sheets with respect to the MD direction of the main body line is not symmetric.

The present invention has therefore been made in view of the aforementioned problems, and has an object of providing a manufacturing method and a manufacturing apparatus for absorbent articles, the manufacturing method and the manufacturing apparatus being able to prevent, without using high level controls, an occurrence of problem caused by placing right and left side flaps not symmetrically with respect to a moving direction (MD direction) of a main body line.

### DISCLOSURE OF THE INVENTION

An aspect of the invention is summarized as a manufacturing method for absorbent articles, each of which includes a front waistline member, a rear waistline member, and a crotch member that connects the front waistline member and the rear waistline member. The manufacturing method for the absorbent articles includes: placing latch members that can be attached to and locked in predetermined regions, on both sides of a center line extending along a moving direction of a flap line, on a flap continuum having a long shape and being continuously transported on the flap line; forming flaps on the flap line, by cutting the flap continuum along a direction crossing the moving direction of the flap line; and placing each of the flaps on an absorbent article continuum having a long shape and being continuously transported on a main body line, so that the center line of the flap continuum extends along a moving direction of the main body line. On the flap line, both side edge portions of the flap continuum are folded back toward the center line .

As described above, the present invention can provide a manufacturing method and a manufacturing apparatus for absorbent articles being able to prevent, without using high level controls, an occurrence of problems caused by placing right and left side flaps not symmetrically with respect to a MD direction of a main body line.

### BRIEF DESCRIPTION OF DRAWINGS

[Fig. 1] Fig. 1 is a diagram for illustrating a manufacturing method for absorbent article according to a first embodiment of the present invention.
[Fig. 2] Fig. 2 is a plan view of an absorbent article according to the first embodiment of the present invention.
[Fig. 3] Fig. 3 is a plan view of an unfolded absorbent article according to the first embodiment of the present invention.
[Fig. 4] Figs. 4A and 4B are diagrams for illustrating a manufacturing method for absorbent articles according to a modified example 1 of the present invention.
[Fig. 5] Figs. 5A and 5B are cross sectional views of flaps of folded absorbent articles according to the modified example 1 of the present invention.
[Fig. 6] Fig. 6 is a plan view of an unfolded absorbent article according to the modified example 1 of the present invention.
[Fig. 7] Fig. 7 is a plan view of an unfolded absorbent article according to the modified example 1 of the present invention.
[Fig. 8] Fig. 8 is a diagram for illustrating a manufacturing method for an absorbent article according to a modified example 2 of the present invention.
[Fig. 9] Fig. 9 is a perspective view of a latch member placing structure 210 according to a second embodiment of the present invention.
[Fig. 10] Fig. 10 is a perspective view of a side edge folding back structure 220 according to the second embodiment of the present invention.
[Fig. 11] Fig. 11 is a perspective view of a cutting and placing structure 230 according to the second embodiment of the present invention.

### PREFERRED EMBODIMENTS FOR CARRYING OUT THE INVENTION

### [First Embodiment]

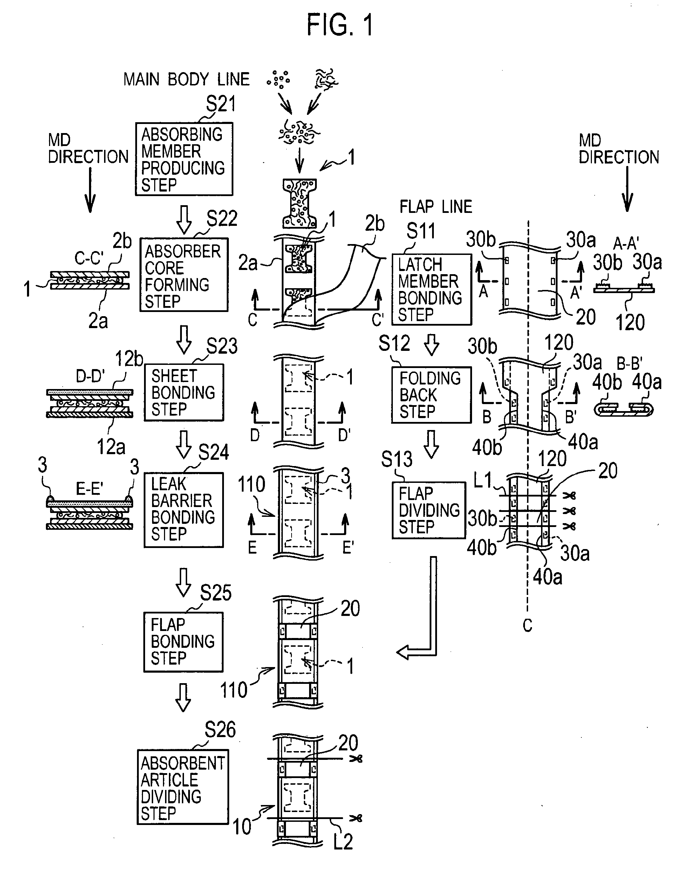

Description will be given of a manufacturing method for absorbent articles according to a first embodiment of the present invention with reference to Figs. 1 to 3.

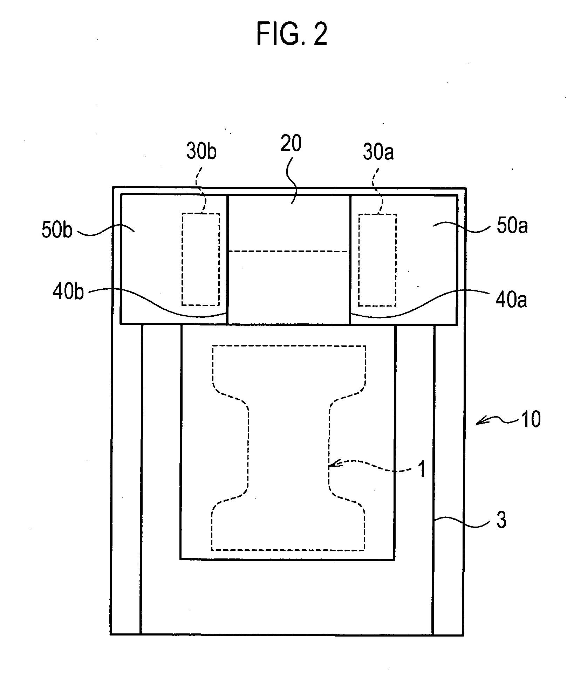

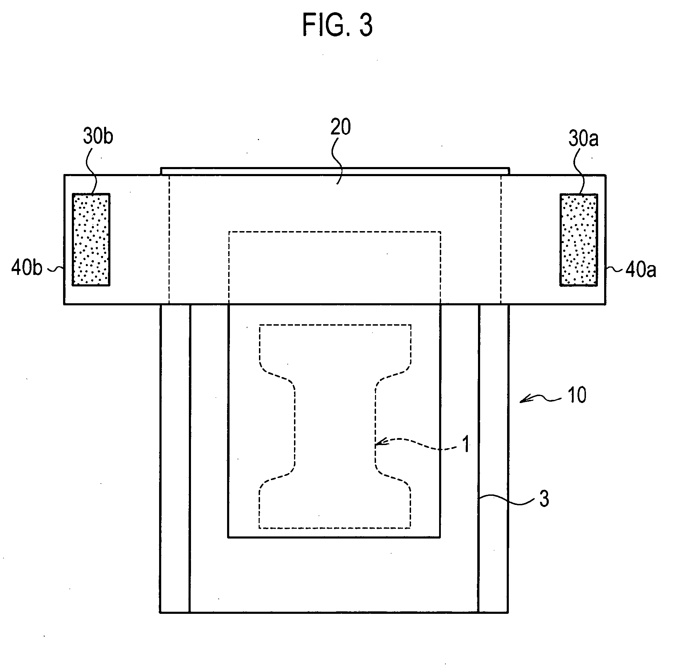

According to the manufacturing method for absorbent articles according to the first embodiment of the present invention, an absorbent article (see Figs. 2 and 3) including a liquid permeable top sheet 12b, a liquid impermeable back sheet 12a and an absorber body interposed between the top sheet 12b and the back sheet 12a, can be manufactured.

Fig. 2 shows an open-type diaper manufactured by the manufacturing method for absorbent articles according to this embodiment. In this open-type diaper, latch members 30a and 30b are locked in and thus temporarily bonded to a main body of a flap 20.

Fig. 3 shows an open-type diaper manufactured by the manufacturing method for absorbent articles according to this embodiment. In this open-type diaper, the latch members 30a and 30b are unlocked from the main body of the flap 20 so that the flap 20 is open.

Hereinafter, with reference to Fig. 1, description will be given of a manufacturing method for absorbent articles according to the present embodiment.

Firstly, steps performed on a flap line, on which the flaps 20 to be placed on the absorbent article are transported, will be described.

In step S11, on the flap continuum 120 having a long shape and being continuously transported on the flap line, latch members 30a and 30b that can be attached to and locked in predetermined regions are placed on both sides of the center line C that extends along the MD direction of the flap line.

Specifically, the latch members 30a and 30b are bonded to both of side edge portions 40a and 40b of the flap continuum 120, by a predetermined interval length.

In this regard, when hook members (male members) are placed as the latch members 30a and 30b, hooked members (female members) are provided as latched members in the predetermined regions of the front waistline member.

Note that, in the present embodiment, the front waistline member is made of a non-woven fabric. Accordingly, the predetermined regions of the front waistline member themselves can serve as the hooked members even when the hooked members are not additionally provided.

In step S12, on the flap line, side edge portions 40a and 40b on both sides of the flap continuum 120 are folded back toward the center line C. Specifically, as shown in the cross-sectional view taken along B-B' line, on the flap line, both of the side edge portions 40a and 40b of the flap continuum 120 are folded back toward the upper side of the flap continuum 120.

In this regard, in order to prevent the flap continuum 120 from being turned over while being transported, the latch members 30a and 30b are locked on the flap continuum 120 and thereby temporarily bonded onto the flap continuum 120. Further, an embossing step or the like may be performed on the folded back portions, to be temporarily bonded onto the flap continuum 120. Here, the folded back portions are referred to as portions formed by the side edge portions 40a and 40b being folded back to cause the flaps 20 to overlap.

In step S13, on the flap line, the flap continuum 120 is cut by the predetermined interval length, in the direction (CD direction) crossing the MD direction of the flap line. Thereby, the flaps 20 are formed.

Secondly, steps performed on a main body line, on which the absorbent article continuum 110 is transported, will be described.

In step S21, a crusher crushes a pulp sheet to produce crushed pulp. Thereafter, when the crushed pulp passes through a pulp supply duct, a super absorbent polymer (SAP) is mixed thereto.

Then, the mixture of the crushed pulp and the super absorbent polymer, which is supplied from the pulp supply duct, is layered in a predetermined shape by use of a layer drum, thereby each of an absorber core 1 is formed.

In step S22, on sheet tissues 2a being continuously transported in the MD direction of the absorber line, each of the absorber cores 1 is placed by a predetermined interval length in the MD direction (moving direction) of the main body line.

Subsequently, on the sheet tissues 2a, sheet tissues 2b being continuously transported in the MD direction of the absorber line are layered. Here, on the sheet tissues 2a,absorber cores 1 are placed along the MD line of the absorber line.

In step S23, top sheets (liquid permeable) 12b being continuously transported in the MD direction of the main body line is placed on and bonded to the sheet tissues 2b. In addition, back sheets (liquid impermeable) 12a being continuously transported in the MD direction of the main body line is placed under and bonded to the sheet tissues 2a.

In step S24, leak barriers 3 formed of different members are bonded to both side edges of the absorber body 10 in the MD direction of the main body line.

In step S25, on the absorbent article continuum having a long shape and being transported on the main body line, the flaps 20 each formed in step S13 is placed so that the center line C of the flaps 20 extends along the MD direction of the main body line.

Specifically, each of the flaps 20 formed on the flap line in step S13 is bonded to the absorbent article continuum 110, by use of a thermoplastic resin.

In this regard, the thermoplastic resin may be entirely applied on the back side of the flaps 20, or the thermoplastic resin may be applied on only a part of the back side of the flaps 20.

Further, in step S25, the flaps 20 may be bonded to the absorbent article continuum 110, by use of a thermo compression or the like, using an embossing roller or a ultrasonic wave.

In step S26, on the main body line, the absorbent article continuum 110 is cut by a predetermined interval length, in the CD direction of the main body line.

According to the manufacturing method for the absorbent articles of the first embodiment of the present invention, it possible to prevent, without using high level controls, an occurrence of problems caused by placing right and left side flaps not symmetrically with respect to the MD direction of the main body line.

Specifically, in the manufacturing method for absorbent articles according to the first embodiment of the present invention, the flaps 20 each having an integrated form serving as right and left side flaps is placed on the absorbent article continuum 110. Accordingly, the right and left side flaps are symmetrically placed with respect to the MD direction of the main body line.

Moreover, in the manufacturing method for absorbent article according to the first embodiment of the present invention, a portion other than the folded portions including the side edge portions 40a and 40b of the flaps 20 can be used as a bonding region between the flaps 20 and the absorbent article continuum 110. Here, the folded back portions are referred to as portions formed by the side edge portions 40a and 40b being folded back to cause the flaps 20 to overlap. Accordingly, this method can maintain high bonding strength between the flaps 20 and the absorbent article continuum 110 while preventing a problem that the thermoplastic resin flows out of the bonding region, which occurs when the bonding regions are small.

In addition, in the manufacturing method for absorbent articles according to the first embodiment of the present invention, on the main body line, each of the flaps 20 is attached to and locked on the folded portions of the absorbent article continuum 110. Accordingly, it is possible to prevent the flaps 20 from being turned over while being transported on the main body line.

According to the manufacturing method for the absorbent articles according to the first embodiment of the present invention, an absorbent article having one pair of side flaps protruded from the absorber body can be manufactured without limitation in the product forms.

### (Modified example 1)

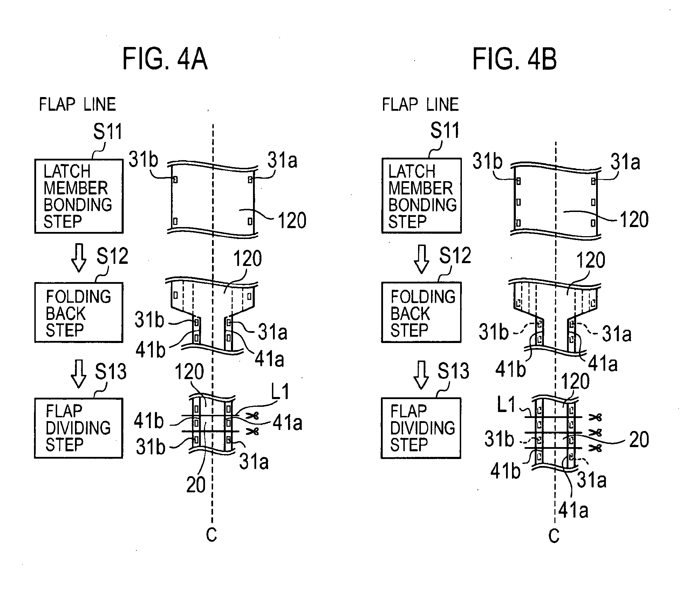

With reference to Figs. 4 to 7, description will be given of a manufacturing method for absorbent articles according to a modified example 1 of the present invention. Hereinafter, the manufacturing method for absorbent articles according to the modified example 1 of the present invention will be described mainly focusing on the differences from the manufacturing method for absorbent article according to the first embodiment of the present invention.

In this manufacturing method for absorbent articles according to the modified example 1, in step S12, the side edge portions 41 a and 41 b of the flap continuum 120 are folded back several times on the flap line.

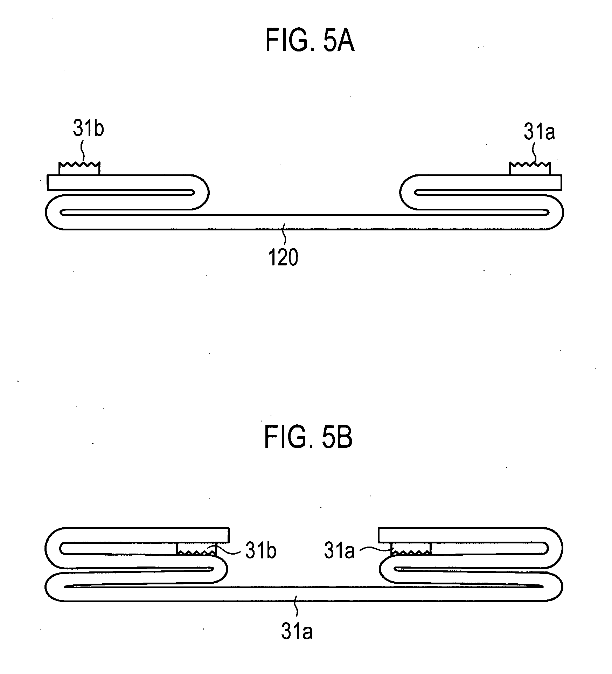

For example, as shown in Fig. 4A, in step S 12, when the side edge portions 41 a and 41 b of the flap continuum 120 are folded back twice on the flap line, latch members 31 a and 31 b face upward as shown in Fig. 5A. In this case, the latch members 31 a and 31 b cannot be locked in the flap continuum 120. Thus, the side edge portions 41 a and 41 b may be temporarily bonded to the flap continuum 120 by a method such as a thermo-compression bonding using an embossed roller or an ultrasonic wave.

Meanwhile, as shown in Fig. 4B, in step S 12, when the side edge portions 41 a and 41 b of the flap continuum 120 are folded back three times on the flap line, the latch members 31 a and 31 b face downward as shown in Fig. 5B. Accordingly, the latch members 31 a and 31 b can be locked on the flap continuum 120. Even in this case, the flaps 20 may be temporarily bonded to the flap continuum 120 by a method such as the thermo-compression bonding using the embossed roller or the ultrasonic wave.

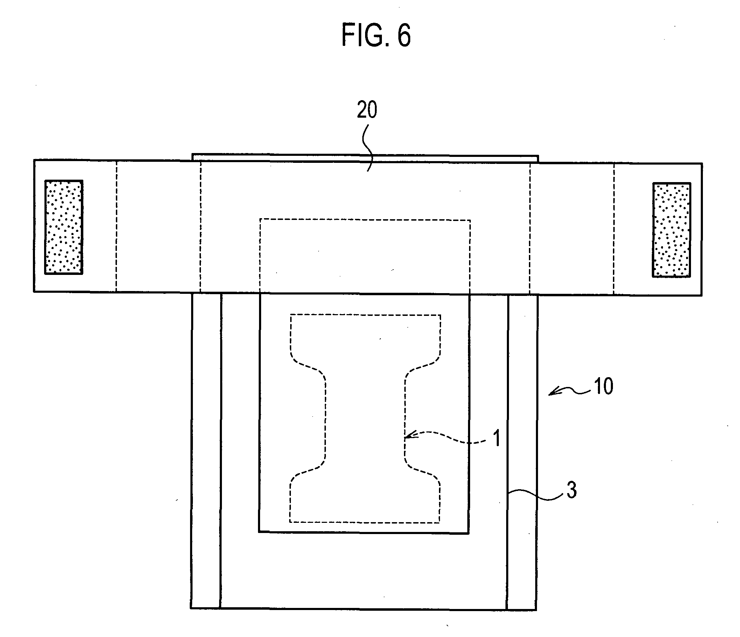

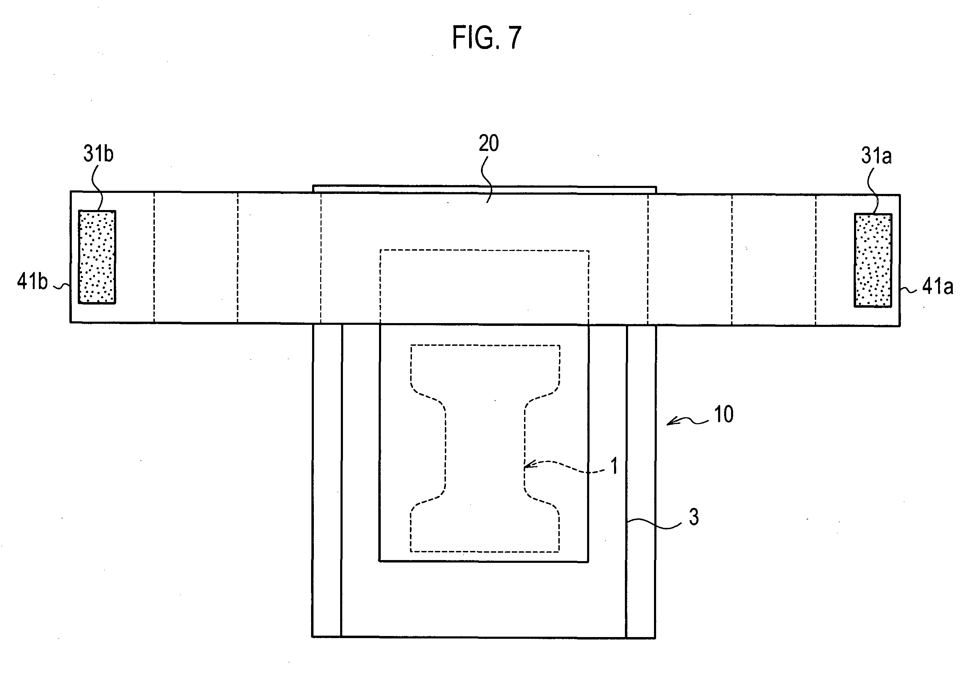

Fig. 6 is a development view of an absorbent article manufactured when the side edge portions 41 a and 41 b of the flap continuum 120 are folded back twice on the flap line in step S12. Fig. 7 is a development view of an absorbent article manufactured when the side edge portions 41 a and 41 b of the flap continuum 120 are folded back three times on the flap line in step S12.

According to the manufacturing method for the absorbent articles in the modified example 1, it is possible to secure the each lengths of the flaps 20 in the CD direction of the main body line longer than the length of the absorber body 10 in the CD direction of the main body line.

### (Modified example 2)

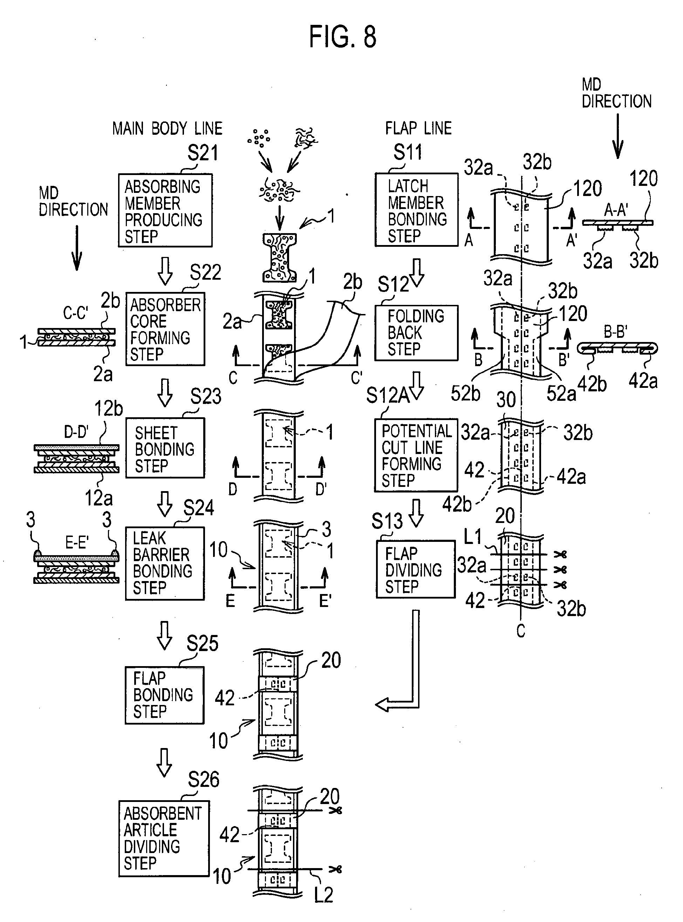

With reference to Fig. 8, description will be given of a manufacturing method for absorbent articles according to a modified example 2 of the present invention. Hereinafter, the manufacturing method for absorbent articles according to the modified example 2 of the present invention will be described mainly focusing on the differences from the manufacturing method for absorbent articles according to the first embodiment of the present invention.

Firstly, steps performed on a flap line will be described.

In step S11, on the flap continuum 120 having a long shape and being continuously transported on the flap line, latch members 30a and 30b that can be attached to and locked in predetermined regions are placed respectively on both sides of the center line C that extends along the MD direction of the flap line.

Specifically, on the flap continuum 120, the latch members 30a and 30b are placed by a predetermined interval length, on both sides of a vicinal region of the center line C that extends along the MD direction of the flap line.

In step S12, on the flap line, side edge portions 42a and 42b of the flap continuum 120 are folded back toward the center line C. Specifically, as shown in the cross-sectional view taken along B-B' line, on the flap line, the side edge portions 42a and 42b of the flap continuum 120 are folded back on the upper side of the flap continuum 120.

In this regard, in order to prevent the flap continuum 120 from being turned over while being transported, an embossing step or the like may be performed on the folded back portions, so that both of the side edge portions 42a and 42b can be temporarily bonded to the flap continuum 120. Here, the folded back portions are referred to as portions formed by the side edge portions 42a and 42b being folded back to cause the flaps 20 to overlap.

In step S12A, on the flap continuum 120, a potential cut line (perforated line) 42 that halves the flap continuum 120 is formed along a direction (CD direction) crossing the MD direction of the flap line.

In step S13, on the flap line, the flap continuum 120 is cut by the predetermined interval length, in the direction (CD direction) crossing the MD direction of the flap line. Thereby, the flaps 20 are formed.

Note that, in step S25 on the main body line, each the flaps 20 formed in the step S13 is vertically turned back. Thereafter, each of the flaps 20 is bonded to the absorbent article continuum 110 by use of a thermoplastic resin.

### [Second Embodiment]

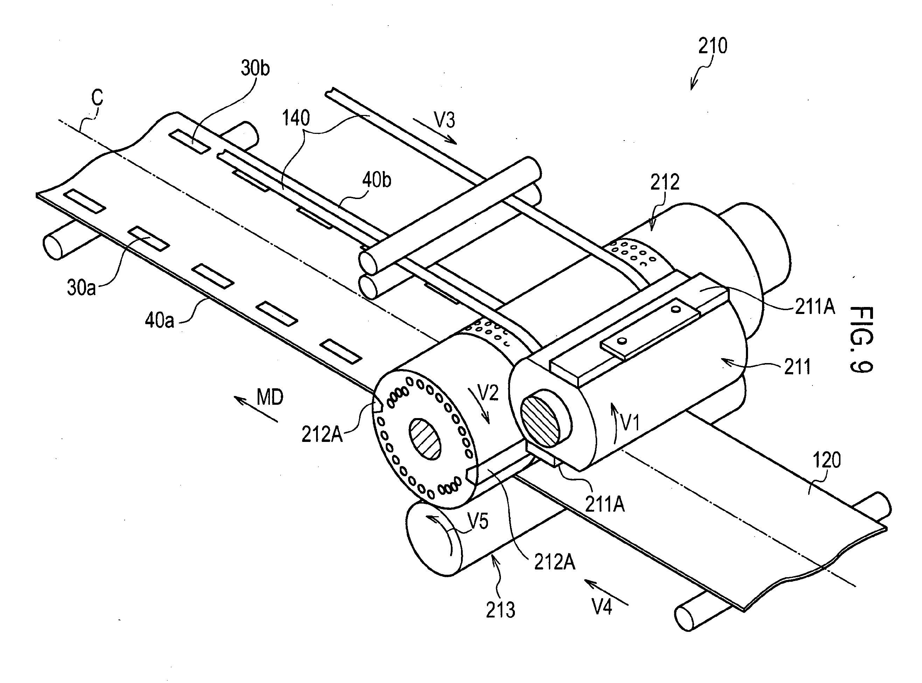

Hereinbelow, a manufacturing apparatus for absorbent articles according to the second embodiment will be described by referring to Figs. 9 to11. The manufacturing apparatus for the absorbent articles is provided with: a latch member placing structure 210; a side edge folding back structure 220;and a cutting and placing structure 230.

First, the latch member placing structure 210 according to the second embodiment will be described by referring to the accompanying drawings. Fig. 9 is a perspective view of the latch member placing structure 210 according to the second embodiment of the present invention.

As shown in Fig. 9, on the flap line (the above-described step S11), the latch member placing structure 210 places the latch members 30a and 30b that can be attached to and locked in a predetermined region, by a predetermined interval length, on both sides of the center line C that extends along the MD direction of the flap line. In the present embodiment, the latch member placing structure 210 places the latch members 30a and 30b on both of the side edge portions 40a and 40b of the flap continuum 120.

Specifically, the latch member placing structure 210 is mainly provided with: an upper blade roll 211, a lower blade roll 212, and a transcript roll 213. Note that, the upper blade roll 211, the lower blade roll 212, and the transcript roll 213 are configured to rotate by using a shaft (not shown) as a center, respectively.

The upper blade roll 211 includes two blades 211 A for cutting the latch member continuum 140. The circumferential velocity (V₁) of the upper blade roll 211 is substantially same as the circumferential velocity (V₂) of the lower blade roll 212.

In addition, the circumferential velocity (V₁) of the upper blade roll 211 is faster than the feeding velocity (V₃) in which the driving roll (not shown) feeds the latch member continuums 140 to the lower blade roll 212. The circumferential velocity (V₁) of the upper blade roll 211 is substantially same as or slower than the moving velocity (V₄) of the flap continuum 120 that is continuously transported by the driving roll (not shown).

The lower blade roll 212 includes two stationary blades 212A that contacts with the blade 211 A so as to cut the latch member continuum 140. The lower blade roll 212 is provided with a plurality of suction holes 212B configured to suck the latch member continuum 140. The lower blade roll 212 rotates in a direction opposite to that of the upper blade roll 211. As described above, the circumferential velocity (V₂) of the lower blade roll 212 is substantially same as the circumferential velocity (V₁) of the upper blade roll 211.

Between the lower blade roll 212 and the transcript roll 213, the transcript roll 213 transcripts (attaches) the latch members 30a and 30b cut by the upper blade roll 211 and the lower blade roll 212, on the flap continuum 120.

The circumferential velocity (V₅) of the transcript roll 213 is substantially same as the moving velocity (V₄) of the flap continuum 120. In other words, the circumferential velocity (V₅) of the transcript roll 213 is substantially same as, or faster than, the circumferential velocity (V₂) of the lower blade roll 212 and the circumferential velocity (V₁) of the upper blade roll 211.

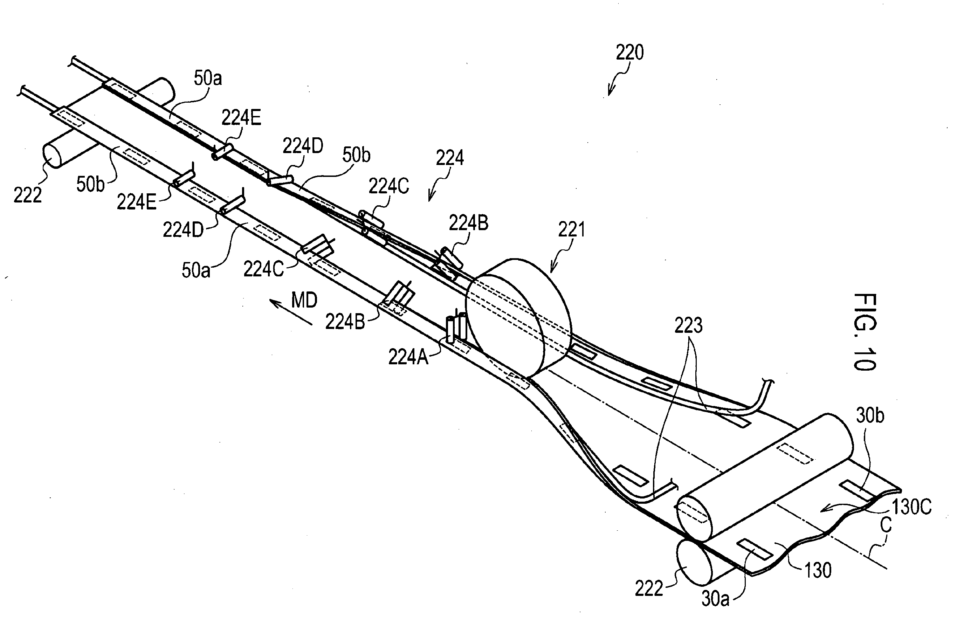

Next, the side edge folding back structure 220 according to the second embodiment will be described by referring to the accompanying drawings. Fig. 10 is a perspective view of the side edge folding back structure 220 according to the second embodiment of the present invention.

As shown in Fig. 10, the side edge portions 40a and 40b placed on both sides of the flap continuum 120 are folded back toward the center line C side.

The side edge folding back structure 220 is mainly provided with: a large diameter roll 221, a transporting roll 222, a folding center bar 223, and a guiding roll 224. Note that, the large diameter roll 221, the transporting roll 222, the folding center bar 223, and the guiding roll 224 are configured to rotate by using a shaft (not shown) as a center, respectively.

The large diameter roll 221 contact with the center region 120C of the flap continuum 120 (that is, center line C side with respect to both of the side edge portions 40a and 40b), and cause, along with the guiding roll 224 to be described later, the side edge portions 40a and 40b to stand.

The transporting roll 222 transports the flap continuum 120 in a substantially horizontal state. In other words, the flap continuum 120 is transported in a substantially horizontal state by the transporting roll 222.

The folding center bar 223 holds the folding back position, when the side edge portions 40a and 40b are folded back. The folding center bar 223 extends in the MD direction, and is placed in substantially parallel with the flap continuum 120 that is transported by the transporting roll 222.

The guiding roll 224 guides the side edge portions 40a and 40b so as to fold back the side edge portions 40a and 40b toward the center line C side. The guiding roll 224 is provided with a plurality of guiding rolls 224A to 224E.

The side edge portions 40a and 40b are caused to stand and to folded back via the folding center bar 223, gradually from the vicinity of the guiding roll 224A to the vicinity of the guiding roll 224E. In other words, the inclined angle of the side edge portions 40a and 40b with respect to the center region 120C of the flap continuum 120 becomes gradually small, from the vicinity of the guiding roll 224A to the vicinity of the guiding roll 224E.

In this regard, in order to prevent the flap continuum 120 from being turned over while being transported, after the side edge portions 40a and 40b are folded back, an embossing step or the like may be performed on the folded back portions including side edge portions 40a and 40b so as to be temporarily bonded.

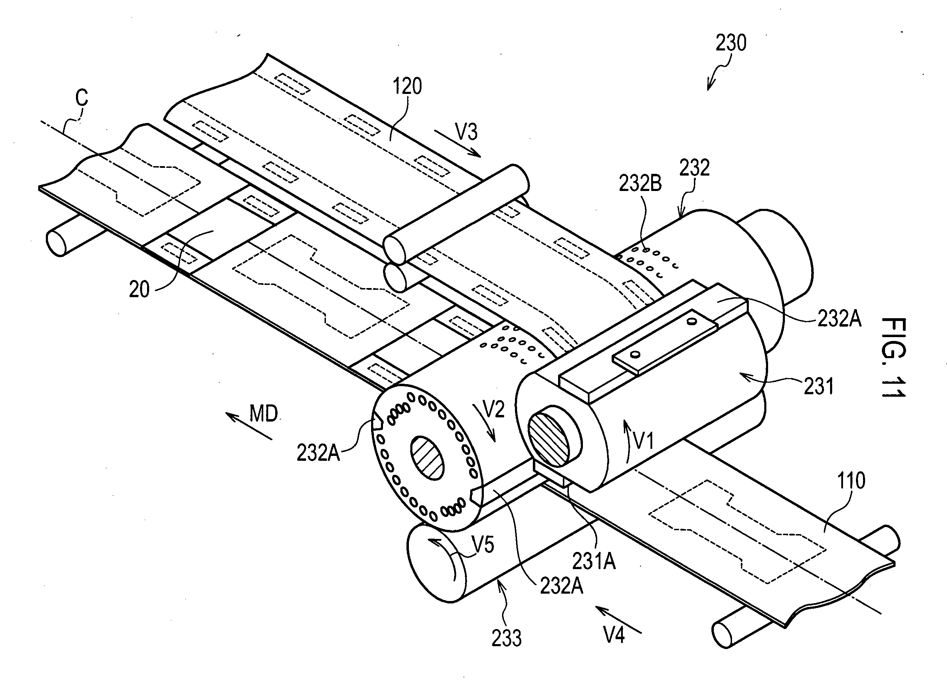

Next, the cutting and placing structure 230 according to the second embodiment will be described by referring to the accompanying drawings. Fig.11 is a perspective view of the potential cut line forming structure 230 according to the second embodiment of the present invention.

As shown in Fig. 11, the cutting and placing structure 230 cuts the flap continuum 120 along the center line in the CD direction of the flap line so as to form the flaps 20. Further, the cutting and placing structure 230 places the flaps 20 by a predetermined interval length on the absorbent article continuum 110, so that the center line C extends in the MD direction of the main body line.

The cutting and placing structure 230 is mainly provided with: an upper blade roll 231, a lower blade roll 232 and a transcript roll 233. The upper blade roll 231 is provided with two blades 231 for cutting the flap continuum 120. The lower blade roll 232 is provided with two stationary blade 232A that contact with the blade 231 A so as to cut the flap continuum 120. The lower blade roll 232 is provided with a plurality of suction holes 232B for sucking the flap continuum 120.

Note that, the configurations of the upper blade roll 231, the lower blade roll 232, and the transcript roll 233 are same as those in the above-described latch member placing structure 210, thereby descriptions thereof will be omitted.

### [Other Embodiments]

It is a matter of course that orders of performing the steps in the above-described manufacturing method for the absorbent articles is not limited to those described above as long as the absorbent article can be manufactured, and the steps to be performed can be appropriately selected in accordance with the intended purpose.

As described above, the present invention has been described in detail by using the above-described embodiments. However, it is obvious for a person skilled in the art that the present invention is not limited to the embodiments described in this specification. The present invention can be implemented as a modification and an amended embodiment without departing from the content and scope of the present invention which is defined by the description of the scope of claims. Accordingly, the description of the present invention is intended to give description as an example and does not have any meaning to limit the present invention.

This application is based upon and claims the benefit of priority from the prior Japanese Patent Application No. JP 2008-094112, filed on March 31, 2008, and Japanese Patent Application No. JP 2009-074770, filed on March 25, 2009, the entire contents of which are incorporated herein by reference.

### INDUSTRIAL APPLICABILITY

As described above, according to the manufacturing method for absorbent articles of the present invention, there can be provided the manufacturing method being able to prevent, without using high level controls, an occurrence of problem caused by placing right and left side flaps not symmetrically with respect to a moving direction (MD direction) of a main body line.

## Claims

1. A manufacturing method for absorbent articles each including front waistline members, rear waistline members, and crotch members, the crotch members connecting each of the front waistline members and the rear waistline members, comprising:
placing latch members that can be attached to and locked in predetermined regions, on both sides of a center line of a flap line extending along a moving direction of the flap line, on a flap continuum having a long shape and being continuously transported on the flap line;
forming flaps on the flap line, by cutting the flap continuum along a direction crossing the moving direction of the flap line; and
placing each of the flaps on an absorbent article continuum having a long shape and being continuously transported on a main body line, so that the center line extends along a moving direction of the main body line; wherein
on the flap line, both side edge portions of the flap continuum are folded back toward the center line .

2. The manufacturing method for the absorbent articles according to claim 1, wherein,
in placing the latch members, the latch members are placed on the both side edge portions of the flap continuum, and
the both edge portions of the flap continuum are folded back toward the center line.

3. The manufacturing method for the absorbent articles according to claim 2, wherein
the both edge portions of the flap continuum are folded back toward the center line, and the latch members placed on the both edge portions are attached to and locked on the flap continuum.

4. The manufacturing method for the absorbent articles according to claim 1, wherein,
in placing the latch members on the flap continuum, the latch members are placed on both sides of a vicinal region of the center line extending along the moving direction of the flap line, and further comprising:
forming a potential cut line that halves the flap continuum along the center line.

5. The manufacturing method for absorbent article according to claim 1,
wherein,
the both edge portions of the flap continuum are folded back toward the center line, several times.

6. The manufacturing method for the absorbent articles according to claim 1, wherein,
in placing the flaps, the flaps are bonded onto the absorbent article continuum.

7. A manufacturing apparatus for absorbent articles each including front waistline members, rear waistline members, and crotch members, the crotch members connecting each of the front waistline members and each of the rear waistline members, comprising:
a latch member placing structure configured to place latch members that can be attached to and locked in predetermined regions, on both sides of a center line extending along a moving direction of a flap line, on a flap continuum having a long shape and being continuously transported on the flap line;
a side edge folding back structure configured to fold back both side edge portions of the flap continuum toward the center line; and
a cutting and placing structure configured to form flaps by cutting the flap continuum along a direction crossing the moving direction of the flap line, and to place each of the flaps on an absorbent article continuum having a long shape, so that the center line extends along a moving direction of a main body line.

## Patentansprüche

1. Herstellungsverfahren für saugfähige Artikel, von denen jeder die vorderen Taillenelemente, hinteren Taillenelemente und Schnittelemente beinhaltet, wobei die Schnittelemente jede der vorderen Taillenelemente und hinteren Taillenelemente verbindet, was Folgendes umfasst:
Platzieren der Greifelemente, die an einem vorbestimmten Bereich angebracht und fixiert werden kann, auf beiden Seiten einer Mittellinie einer Laschenlinie, die sich an einer Bewegungsrichtung der Laschenlinie erstreckt, auf einem Laschenkontinuum, das eine lange Form hat und kontinuierlich auf der Laschenlinie befördert wird,
Bilden der Laschen auf der Laschenlinie, indem das Laschenkontinuum in einer Richtung, die die Bewegungsrichtung der Laschenlinie überquert, geschnitten wird, und
Platzieren jeder der Laschen auf ein saugfähiges Artikelkontinuum, das eine lange Form hat und kontinuierlich auf einer Hauptkörperlinie transportiert wird, sodass sich die Mittellinie in Bewegungsrichtung der Hauptköperlinie erstreckt, wobei
auf der Laschenlinie beide Seitenrandabschnitte des Laschenkontinuums in Richtung der Mittellinie zurückgefaltet sind.

2. Herstellungsverfahren für die saugfähigen Artikel nach Anspruch 1, wobei
beim Platzieren der Greifelemente die Greifelemente auf beiden Seitenrandabschnitten des Laschenkontinuums platziert werden, und
die beiden Randabschnitte des Laschenkontinuums in Richtung der Mittellinie zurückgefaltet werden.

3. Herstellungsverfahren für die saugfähigen Artikel nach Anspruch 2, wobei
die beiden Randabschnitte des Laschenkontinuums in Richtung der Mittellinie zurückgefaltet werden, und die Greifelemente, die an beiden Randabschnitten platziert werden, am Laschenkontinuum angebracht und ergriffen werden.

4. Herstellungsverfahren für die saugfähigen Artikel nach Anspruch 1, wobei
durch Platzieren der Greifelemente am Laschenkontinuum die Greifelemente auf beiden Seiten eines benachbarten Bereichs der Mittellinie platziert werden, die sich entlang der Bewegungsrichtung der Laschenlinie erstreckt und ferner Folgendes umfasst:
Bilden einer potenziellen Schnittlinie, die das Laschenkontinuum an der Mittellinie entlang halbiert.

5. Herstellungsverfahren für saugfähige Artikel nach Anspruch 1, wobei
beide Randabschnitte des Laschenkontinuums in Richtung der Mittellinie mehrere Male zurückgefaltet werden.

6. Herstellungsverfahren für absorbierende Artikel nach Anspruch 1, wobei
durch Platzieren der Laschen die Laschen mit dem saugfähigen Artikelkontinuum verbunden werden.

7. Herstellungsgerät für saugfähige Artikel, von denen jeder die vorderen Taillenelemente, hinteren Taillenelemente und Schnittelemente beinhalten, wobei die Schnittelemente jede der vorderen Taillenelemente und jeden der hinteren Taillenelemente verbindet, die Folgendes umfassen:
eine Struktur zum Platzieren des Greifelements, das so konfiguriert ist, dass die Greifelemente, die in vorbestimmten Bereichen angebracht und gesperrt werden können, auf beiden Seiten der Mittellinie, die sich in einer Bewegungsrichtung einer Laschenlinie erstreckt, auf einem Laschenkontinuum, das einer lange Form hat und kontinuierlich auf der Laschenlinie befördert wird:
eine Struktur zum Zurückfalten des Seitenrands, die so konfiguriert ist, dass sich beide Seitenrandabschnitte des Laschenkontinuums in Richtung der Mittellinie zurückfalten, und
eine Struktur zum Schneiden und Platzieren, die so konfiguriert ist, dass sie Laschen durch Schneiden der Laschenkontinuums an einer Richtung entlang bildet, die die Bewegungsrichtung der Laschenlinie überquert, und das Platzieren jeder der Laschen auf ein saugfähiges Artikelkontinuum, das eine lange Form hat, sodass sich die Mittellinie in einer Bewegungsrichtung einer Hauptkörperlinie erstreckt.

## Revendications

1. Procédé de fabrication d'articles absorbants comprenant chacun des membres avant de taille, des membres arrière de taille et des membres d'entrejambe, les membres d'entrejambe se raccordant à chacun des membres avant de taille et des membres arrière de taille, comprenant :
poser des membres de fermeture qui peuvent être attachés à et bloqués dans des régions prédéterminées, des deux côtés d'une ligne médiane d'une ligne de rabat s'étendant le long d'une direction de déplacement de la ligne de rabat, sur un continuum de rabat ayant une forme longue et étant transporté continuellement sur la ligne de rabat;
former des rabats sur la ligne de rabat, en coupant le continuum de rabat le long d'une direction croisant la direction de déplacement de la ligne de rabat; et
poser chacun des rabats sur un continuum d'article absorbant ayant une forme longue et étant transporté continuellement sur une ligne de corps principal, de sorte que la ligne médiane s'étend le long d'une direction de déplacement de la ligne de corps principal; dans lequel
sur la ligne de rabat, les deux parties de bord latéral du continuum de rabat sont rabattues vers la ligne médiane.

2. Procédé de fabrication d'articles absorbants selon la revendication 1, dans lequel :
en posant les membres de fermeture, les membres de fermeture sont posés sur les deux parties de bord latéral du continuum de rabat, et
les deux parties de bord du continuum de rabat sont rabattues vers la ligne médiane.

3. Procédé de fabrication d'articles absorbants selon la revendication 2, dans lequel :
les deux parties de bord du continuum de rabat sont rabattues vers la ligne médiane et les membres de fermeture posés sur les deux parties de bord sont attachés à et bloqués sur le continuum de rabat.

4. Procédé de fabrication d'articles absorbants selon la revendication 1, dans lequel :
en posant les membres de fermeture sur le continuum de rabat, les membres de fermeture sont posés sur les deux côtés d'une région voisine de la ligne médiane s'étendant le long de la direction de déplacement de la ligne de rabat, et comprenant en outre :
former une ligne de coupe potentielle qui divise en deux le continuum de rabat le long de la ligne médiane.

5. Procédé de fabrication d'articles absorbants selon la revendication 1, dans lequel :
les deux parties de bord du continuum de rabat sont rabattues plusieurs fois vers la ligne médiane.

6. Procédé de fabrication d'articles absorbants selon la revendication 1, dans lequel :
en posant les rabats, les rabats adhèrent au continuum d'article absorbant.

7. Appareil de fabrication d'articles absorbants comprenant chacun des membres avant de taille, des membres arrière de taille et des membres d'entrejambe, les membres d'entrejambe se raccordant à chacun des membres avant de taille et à chacun des membres arrière de taille, comprenant :
une structure de pose de membres de fermeture configurée pour poser des membres de fermeture qui peuvent être attachés à et bloqués dans des régions prédéterminées, des deux côtés d'une ligne médiane s'étendant le long d'une direction de déplacement d'une ligne de rabat, sur un continuum de rabat ayant une forme longue et étant transporté continuellement sur la ligne de rabat;
une structure de rabattement de bord latéral configurée pour rabattre les deux parties de bord latéral du continuum de rabat vers la ligne médiane; et
une structure de coupe et de pose configurée pour former des rabats en coupant le continuum de rabat le long d'une direction croisant la direction de déplacement de la ligne de rabat et pour poser chacun des rabats sur un continuum d'article absorbant ayant une forme longue de telle sorte que la ligne médiane s'étend le long d'une direction de déplacement d'une ligne de corps principal.
